# EUROPEAN PATENT APPLICATION

(11) **EP 4 613 859 A1**
(43) Date of publication of application: **10.09.2025**
(21) Application number: 23886371.6
(22) Date of filing: 03.11.2023
(51) Int. Cl.: C12N 15/10, C07K 14/47, C40B 30/04

(54) **TLR SIGNALING INHIBITORY PEPTIDE AND COMPOSITION INCLUDING SAME FOR PREVENTION OR TREATMENT OF INFLAMMATORY DISEASES**

(30) Priority: 03.11.2022 KR 20220144971
(71) Applicant: Korea Institute of Science and Technology, Seoul 02792 (KR)
(72) Inventor: SEO, Moon-Hyeong, Gangneung-si, Gangwon-do 25451 (KR); LEE, Wook-Bin, Gangneung-si, Gangwon-do 25451 (KR); PARK, Keun-Wan, Gangneung-si, Gangwon-do 25451 (KR); JUNG, Sang-Hoon, Gangneung-si, Gangwon-do 25451 (KR); KIM, Meong-Il, Gangneung-si, Gangwon-do 25451 (KR); LIM, Yun, Gangneung-si, Gangwon-do 25451 (KR)
(74) Representative: Handley, Matthew Edward
(86) International application number: PCT/KR2023/017529
(87) International publication number: WO 2024/096677

(57) **Abstract**

The present invention relates to a peptide library derived from vast TIR domains of different biological species and a construction method thereof, a novel peptide involved in a TLR signaling pathway by binding to human TIR domains screened from the library and a use thereof as a TLR4 signaling inhibitor.

## Description

### [Technical Field]

The present invention relates to a TLR signal-inhibiting peptide which is involved in the interaction between a peptide library constructed from the sequences of TIR-like domains and TIR domains selected therefrom, and a use of the same for preventing or treating an inflammatory disease.

### [Background Art]

Toll-like receptors (TLRs), a family of proteins that recognize external pathogens or materials secreted from damaged or aged tissues in the human body, have a toll/interleukin-1 receptor (TIR) domain in a cell, and homologous/heterologous binding to the TIR domain of an adaptor protein expressed in the cell is a key mechanism for protecting the human body by transmitting an activated signal. Excessive activation and failure to regulate TLR signals may not only be the major cause of chronic inflammatory diseases such as autoimmune diseases and sepsis, but the interaction of TIR domains during viral or pathogenic infection may also be utilized as a target for immune evasion by pathogens. Accordingly, human TIR domain sequence-derived peptide inhibitors have been developed to inhibit TIR-TIR interactions in cells and have shown efficacy in immunomodulation and treatment of inflammatory diseases, but most of them have been obtained only through a low-throughput method using peptide synthesis, which has the limitation of insufficient utilization of the sequences of TIR domains present in different biological species.

Since the TIR domains of different biological species in nature have been reported to have various cross-species (e.g., bacteria-human or bacteria-plant) heterologous bindings, a method is needed to search for peptide inhibitors that can regulate the vast TIR domain sequences in nature, and novel peptide inhibitors that can suppress these TIR-TIR interactions can be used to treat a variety of diseases through immunomodulation.

### [Disclosure]

### [Technical Problem]

The present inventors manufactured approximately 190,000 peptide sequences by designing sequences on the TIR surface from the vast TIR domains of different biological species and constructed a phage display library using the sequences and thus completed the present invention relating to a novel peptide candidate sequence that regulates a TRL signaling pathway by binding to a human TIR domain using the above phage display library and a screening method thereof.

Therefore, the present invention is directed to providing a method of constructing a phage display library of peptides including a surface sequence of a TIR domain.

The present invention is also directed to providing a method of screening a peptide having TLR signaling inhibitory activity, which is derived from the surface sequence of a TIR domain.

The present invention is also directed to providing a TLR signal-inhibiting peptide derived from the surface sequence of a TIR domain.

The present invention is also directed to providing a composition for preventing, improving or treating an inflammatory disease, which includes a TLR signal-inhibiting peptide.

### [Technical Solution]

One aspect of the present invention provides a method of constructing a phage display library of peptides including the surface sequence of a TIR domain.

Another aspect of the present invention provides a method of screening a peptide having TLR signaling inhibitory activity, which is derived from the surface sequence of a TIR domain.

Still another aspect of the present invention provides a TLR signal-inhibiting peptide derived from the surface sequence of a TIR domain.

Yet another aspect of the present invention provides a composition for preventing, improving or treating an inflammatory disease, which includes a TLR signal-inhibiting peptide.

Hereinafter, the present invention will be described in detail.

In one aspect of the present invention, the present invention provides a library of peptides including the surface sequences of TIR domains from the TIR domains of various biological species, and a method of constructing the same.

The peptide library was constructed by a method including the following steps. First, to extract TIR domains from various species of organisms based on cross-species binding of TIR-like domains existing in nature, full sequence alignment for the surface sequences of 13,644 TIR domains (PF01582) from PFAM was used, and a sequence of approximately 16 amino acids was designed based on all surface sequences excluding the core region of the tertiary structure of the TIR domain, thereby constructing a library of 190,946 peptides.

In addition, peptides having TLR inhibitory activity were screened from the constructed peptide library. The screening may be performed using, for example, a phage display technique. A peptide may be genetically linked, inserted or substituted into the coat protein of a phage by the phage display technique and displayed on the outside of the phage, and the peptide may be encoded by the genetic information in a virion.

The term "phage" or "bacteriophage" used herein may be used interchangeably, and may refer to a virus that infects bacteria and is replicated in the bacteria. The phage or bacteriophage may be used to display a peptide included in the library of the present invention, and the peptide may be genetically manipulated to be displayed on the coat protein of the phage or a fragment thereof. The genetic manipulation involves the introduction of a gene encoding the peptide.

The phage "phage display" used herein may refer to the display of a functional foreign peptide or protein on the surface of a phage or phagemid particle. The phage surface may mean the coat protein of a phage or a fragment thereof. In addition, the phage may be a phage in which the C-terminus of the functional foreign peptide is linked to the N-terminus of the coat protein of the phage, or the peptide may be inserted into the sequence of consecutive amino acids of the coat protein of the phage or may replace a part of the sequence of consecutive amino acids of the coat protein thereof. In addition, the coat protein is not limited in type, and may be p3, p6, p8, p9, or a fusion thereof.

To screen peptides having inhibitory activity on a TLR signaling pathway among the phage-displayed peptides, peptides that specifically bind to MAL or MyD88, which is a key adaptor protein for TLR signals, were screened. As a result, a large amount of MAL or MyD88-bidning peptide sequences were obtained through NGS performed on the base sequences encoding the screened peptides.

In addition, in one embodiment of the present invention, it was confirmed that the peptides screened by the phage display method control TLR signals and, in particularly, have the function of inhibiting the signaling pathway.

The screened TIR domain-binding peptides having TLR inhibitory activity may include peptides consisting of the amino acid sequences listed in Tables 1 to 4, but the present invention is not limited thereto, and these peptides may further include a sequence for cell penetration. In one embodiment of the present invention, the TIR domain-binding peptides may further include a peptide sequence for cell penetration. Peptides in which the TIR domain-binding peptides and cell-penetrating peptides are linked may be manufactured as in SEQ ID NOs: 1 to 21, but the present invention is not limited thereto. Cloning was performed using the above-manufactured peptides.

The TLR signaling pathway is a signaling pathway regulated by a toll-like receptor (TLR). While the TLR is a key protein for a defense mechanism of recognizing an external pathogen or damaged or aged tissue in the human body and removing it, excessive activation of TLR signals may be the major cause of severe chronic inflammatory diseases such as infectious diseases, sepsis, autoimmune diseases, and degenerative brain diseases. Additionally, TLRs play an important role in regulating inflammatory diseases, so there are various drugs targeting them.

The TIR domain-binding peptides of the present invention may be utilized as therapeutic agents for various inflammatory diseases due to the TRL signal inhibitory effect. The inflammatory diseases are immune responses triggered by various causes such as infections by pathogens or tissue damage. More specifically, the inflammatory disease of the present specification may be, but not limited to, any one disease selected from the group consisting of dermatitis, allergies, atopy, asthma, periodontitis, allergic and non-allergic rhinitis, otitis media, pharyngitis, tonsillitis, pneumonia, peptic ulcers, chronic and acute gastritis, Crohn's disease, colitis, chronic and acute enteritis, hemorrhoids, gout, ankylosing spondylitis, osteoarthritis, rheumatoid arthritis, periarthritis, tendonitis, tenosynovitis, myositis, acute and chronic hepatitis, Sjogren's syndrome, multiple sclerosis, rhinitis, chronic obstructive pulmonary disease, irritable bowel syndrome, viral infections, bacterial infections, fungal infections, burns, wounds from surgical or dental operations, atherosclerosis, arthritis, Hodgkin's disease, pancreatitis, iritis, scleritis, uveitis, conjunctivitis, dry macular degeneration, wet macular degeneration, sepsis, and septic shock, and preferably, dry macular degeneration, sepsis, or septic shock.

Particularly, the "sepsis" used herein refers to a disease in which a severe inflammatory response occurs throughout the body, and particularly, a disease that can cause serious organ damage to an abnormality in the immune system caused by microbial infection. A condition in which circulatory, cellular, and metabolic abnormalities occur due to worsening of the above symptoms is called septic shock, which is a serious disease with a high mortality rate.

As an embodiment of the present invention, "prevention, improvement, or treatment of sepsis" includes reduction, improvement or removal of all or some symptoms associated with sepsis and conditions associated with multiorgan dysfunction syndrome.

In addition, "macular degeneration" used herein is a chronically progressive degenerative disease of the macula, and is known to be caused by excessive oxygen radicals, mitochondrial dysfunction, inflammation, abnormal lipid metabolism, genetic factors, or environmental factors (smoking or exposure to UV rays), but its exact cause has not been identified. The macular degeneration may be, particularly, dry macular degeneration which accounts for 85 to 90% of all macular degeneration, and late-stage macular degeneration may also lead to wet macular degeneration. Dry macular degeneration is characterized by an increase in medium-sized or larger yellow deposits called drusen, which is present particularly under the retinal pigment epithelium, pigmentary abnormalities in the retinal pigment epithelium, and deposits called reticular pseudodrusen, which is present under the retina, and progressive atrophy of the nerves in the macular area.

As an embodiment of the present invention, "prevention, improvement or treatment of macular degeneration" includes reduction, removal and improvement of symptoms associated with macular degeneration by preventing the formation of waste products in the retinal pigment epithelium (RPE) and the outer nuclear layer (ONL) and the atrophy of photoreceptor cells.

In the present specification, the term "prevention" refers to all actions of inhibiting the disease or delaying the occurrence thereof by the administration of the pharmaceutical composition according to the present invention.

In the present specification, the term "treatment" refers to all actions involved in improving or beneficially changing symptoms of the disease by the administration of the pharmaceutical composition according to the present invention.

The pharmaceutical composition according to the present invention may be used in an oral formulation such as a powder, a granule, a tablet, a capsule, a suspension, an emulsion, a syrup, or an aerosol, a quasi-drug, a suppository, or a sterilized injectable solution according to a conventional method, and may further include a carrier or excipient, which is required for formulation. Pharmaceutically acceptable carriers, excipients and diluents, which may be further added to the active ingredient, include lactose, dextrose, sucrose, sorbitol, mannitol, xylitol, erythritol, maltitol, starch, acacia gum, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, magnesium stearate, and mineral oil. The pharmaceutical composition according to the present invention is formulated using diluents or excipients such as a filler, a bulking agent, a binder, a wetting agent, a disintegrant, and a surfactant, which are generally used in preparation.

For example, solid preparations for oral administration include tablets, pills, powders, granules, and capsules, and such solid preparations are prepared by mixing at least one excipient, such as starch, calcium carbonate, sucrose, lactose, or gelatin with the extract or compound. Aside from simple excipients, lubricants such as magnesium stearate, talc, etc. are further used. Liquid preparations for oral administration include suspensions, liquids for internal use, emulsions, and syrups, and may further include various types of excipients, for example, a wetting agent, a sweetener, a flavoring agent, and a preservative, other than a commonly-used simple diluent such as water, or liquid paraffin.

Preparations for parenteral administration may include sterilized aqueous solutions, non-aqueous solvents, suspensions, emulsions, freeze-dried formulations, or suppositories. As the non-aqueous solvent or suspension, propylene glycol, polyethylene glycol, a vegetable oil such as olive oil, or an injectable ester such as ethyl oleate may be used. As a suppository base, Witepsol, Tween 61, cacao butter, laurin butter, or glycerogelatin may be used.

The pharmaceutical composition of the present invention may be administered orally or parenterally (e.g., intravenously, subcutaneously, intraperitoneally or locally) depending on a desired method, and a dose of the pharmaceutical composition may vary depending on the condition and body weight of a patient, the severity of a disease, a drug type, an administration route and time, and may be suitably selected by one of ordinary skill in the art.

The pharmaceutical composition of the present invention is administered at a pharmaceutically effective amount. The "pharmaceutically effective amount" used herein refers to an amount sufficient for treating a disease at a reasonable ratio applicable for medical treatment, and the amount may be determined by parameters including a type of a patient's disease, severity, drug activity, sensitivity to a drug, administration time, an administration route and an excretion rate, the duration of treatment, ingredients concurrently used, and other factors. The pharmaceutical composition of the present invention may be administered separately or in combination with other therapeutic agents, and may be sequentially or simultaneously administered with a conventional therapeutic agent. Taking all of the above parameters into consideration, a dose can be determined at a level that minimizes side effects, and may be easily determined by one of ordinary skill in the art. Specifically, the dose of the pharmaceutical composition may vary according to a patient's age, weight, severity, or sex, and generally, 0.001 to 150 mg, and preferably, 0.01 to 100 mg per kg of weight may be administered daily or every other day, or one to three times a day. However, the dose is merely an example, and may be determined differently as needed.

### [Advantageous Effects]

According to the present invention, a library of approximately 190,000 peptides derived from TIR surface sequences from the vast TIR domains of various biological species can be constructed to screen novel peptides binding to human TIR domains (MAL and MYD88), and these peptides can be used as TLR signaling inhibitors.

### [Description of Drawings]

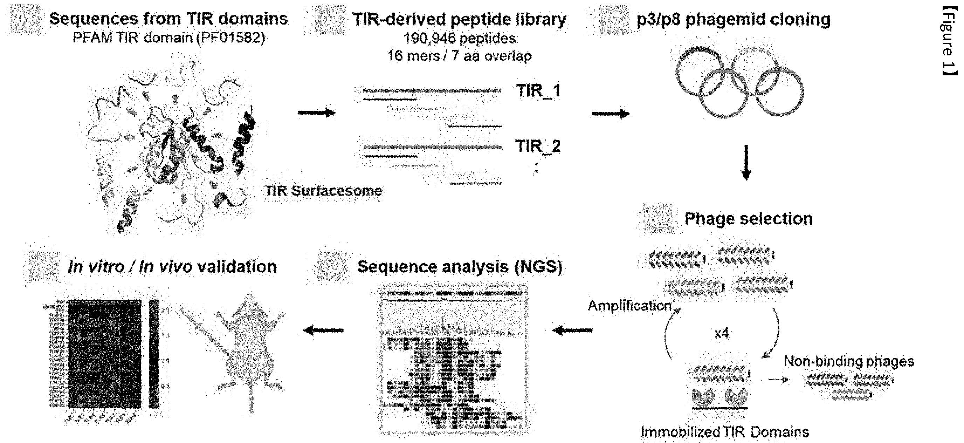

FIG. 1 is a schematic diagram illustrating a process of constructing a library of peptides binding to human TIR domains designed using the entire sequence for a TIR domain in PFAM.
FIG. 2 shows the results of confirming the inhibitory efficacy of a peptide of the present invention on various TRLs.
FIG. 3 shows the results of confirming TLR4 inhibitory activity efficacy by screening peptides that specifically bind to MAL and MyD88.
FIG. 4 shows the results of confirming the concentration-dependent inhibitory effect of peptides with confirmed TRL4 inhibitory activity.
FIG. 5 shows the results of confirming the effect of 4 types of peptides with confirmed TLR4 inhibitory activity on inhibiting the phosphorylation of IRAK4 and IRF3, which are downstream proteins of TLR4 signaling.
FIG. 6 shows the results of confirming the effect of 4 types of peptides with confirmed TLR4 inhibitory activity on inhibiting the phosphorylation of ERK, p38, and JNK, which are downstream proteins of TLR4 signaling.
FIG. 7 shows the results of confirming the NF-kB nuclear localization inhibitory effect of 4 types of peptides with confirmed TRL4 inhibitory activity.
FIG. 8 shows the results of confirming the cytokine secretion inhibition effect of peptides with TRL4 inhibitory activity in macrophages.
FIG. 9 shows the results of confirming the survival rate of mice as a sepsis treatment effect of peptides with TRL4 inhibitory activity.
FIG. 10 shows the results of confirming the cytokine secretion inhibition effect in relation to the sepsis treatment effect of peptides with TRL4 inhibitory activity.
FIG. 11 shows the results of confirming the effect of peptides with TRL4 inhibitory activity on renal cell apoptosis in relation to the sepsis treatment effect.
FIG. 12 shows the results of confirming the effect of protecting the retinal pigment epithelium (RPE) and the outer nuclear layer (ONL) in relation to the dry macular degeneration treatment effect of peptides with TRL4 inhibitory activity by SD-OCT and H&E staining.
FIG. 13 show the results of quantitatively analyzing the thicknesses of the RPE and the ONL, observed in FIG. 12.

### [Best modes of the Invention]

Hereinafter, the present specification will be described in detail with reference to examples. However, the examples according to the present specification may be modified into a variety of different forms, and it should not be construed that the scope of the present specification is limited to the following examples. The examples of the present specification are provided to more completely explain the present specification to those of ordinary skill in the art.

### Example 1. Construction of library of TIR domain-binding peptides

To extract TIR domains present in various species of organisms, full sequence alignment (#13644) for the surface sequences of 13,644 TIR domains (PF01582) from PFAM was used.

16-mer amino acid sequences (187,814) were designed by being cleaved to overlap approximately 7 amino acids across all the surface sequences excluding the core region of the tertiary structure of the TIR domains belonging to the biological species, and additionally, a list of 3,132 peptide sequences were constructed with surface sequences of 14 types of human TIR domains, 2 types of bacterial TIR domains, and 3 types of non-TIR domains known to bind to TIR. As a result, a library of approximately 190,946 16-mer peptides was constructed.

### Example 2. Screening of peptides with TLR inhibitory activity

### 2-1. Phage display using phagemid

To screen peptides having TLR inhibitory activity from the peptide library constructed in Example 1, phage display was performed as follows.

First, vector cloning was performed by synthesizing a DNA sequence for expressing each peptide belonging to the library constructed in Example 1. Vector cloning was performed using p3/p8 phagemids. The vector was introduced into an M13 bacteriophage, and the peptide derived from a TIR domain was displayed on the surface of the phage.

### 2-2. Confirmation of TLR signaling pathway inhibitory activity

Peptides that specifically bound to key proteins of the TLR signaling pathway, such as MAL and MyD88, to inhibit their activity were screened from the phage-displayed peptides obtained in 2-1. A total of four rounds of a process, which includes immobilizing MAL and MyD88 proteins produced in *E. coli* on a 96-well plate, fusing the proteins to a P8 or P3 coat protein and then mixing the phage-displayed peptide library with the fused proteins, and amplifying the remaining phages in *E. coli* after removing non-binding phages and then re-binding the amplified results, were performed. After the fourth round, the remaining phages were obtained by binding to the finally immobilized target proteins, and peptide sequences binding to the target and the relative distribution of each sequence were confirmed by performing NGS analysis on phagemids in the phages.

Approximately 500 peptide sequences binding to each of the human MAL and MYD88 domains were screened using the library which had been phage-displayed using the P8 or P3 coat protein, and the top 30 peptide sequences are shown in Tables 1 to 4 below.

**[Table 1]**

| **P8_MAL** | | |
|---|---|---|
| **Peptide sequence** | **NGS count** | **Population** |
| WFYERLVSLILRDTKV | 79000 | 24.40% |
| AFLYTFLINLTHPTNF | 51063 | 18.86% |
| AFFWAQLKSVLGNPTM | 28801 | 8.90% |
| RNFVAYLFQALANKGI | 27950 | 8.63% |
| TFLGHLYNALVQAGIH | 24732 | 7.64% |
| LFFWESLKQLLRSDGR | 22560 | 5.97% |
| YFLHFLLFSRPETEYI | 17198 | 5.31% |
| AFFWEQLRTVLGKPTM | 11826 | 3.65% |
| QLIQQWIELHLHQLHL | 9394 | 2.90% |
| DTKLKKGESIAPELLR | 5617 | 1.74% |
| KLFWEKFKEFFLPSLSR | 4888 | 1.51% |
| SEFIQKIILWMNPKIV | 4458 | 1.38% |
| HFFWIQLKSILGETSF | 3624 | 1.12% |
| WFLFTVRSAIYAMSPA | 3156 | 0.97% |
| RNNFIGHLYQALYRIG | 3120 | 0.96% |
| SLLYDHLRWLGIRAFL | 2576 | 0.80% |
| WFMMKFRTVLDHVNDV | 2269 | J.70% |
| KLIQGWFLYRLLKIEQ | 2068 | J.64% |
| DFLNWFIALKSTVIEN | 867 | J.27% |
| NQWRNALTAAANLSGW | 848 | J.26% |
| FTLAVHVRNWLVGGFI | 520 | J.19% |
| YLKWGYPWFWDRLRFA | 517 | J.19% |
| AYANFRQGLLRLLELA | 466 | J.14% |
| WFLTAFRMAMDQVADT | 353 | J.11% |
| YLISYIFNELARAGFS | 295 | 0.09% |
| AWALHQLQQIINWHET | 232 | 0.07% |
| WALQELVKILQAKIFL | 227 | 0.07% |
| KFIWDIINAISSQIRV | 146 | J.05% |

**[Table 2]**

| **P3_MAL** | | |
|---|---|---|
| **Peptide sequence** | **NGS count** | **Population** |
| YAFTGHLHRYLVQRGV | 43764 | 16.44% |
| SSFREGLWRLLELSKK | 29955 | 11.26% |
| PNLRTKVLNYLMRTKT | 26931 | 10.12% |
| NQWRNALTAAANLSGW | 25831 | 9.71% |
| RGFLSHLRHHLQAKDH | 15065 | 5.66% |
| RLLKWKEALHQFHSWV | 14924 | 5.61% |
| LKVESWRQALEKAGKL | 5180 | 2.32% |
| LKWGDPYFWSKLRYAM | 5128 | 1.93% |
| DWFLSKLSQHILKREK | 4580 | 1.72% |
| KMQKWKDALYEAANLS | 4427 | 1.66% |
| KEVEGWRDALTKVASL | 3758 | 1.41% |
| ESILNQLRRSSQSIAN | 3680 | 1.38% |
| SLRKNLVSNLIEEFKR | 3569 | 1.34% |
| KNLFNELVSKNIRTFM | 3259 | 1.22% |
| HHKGSYGEALAEHEKR | 3056 | 1.15% |
| FLNFRGLDVRNYFLGH | 2855 | 1.07% |
| KWFWDKLIYALSRNPH | 2796 | 1.05% |
| YNFISHLYDALHRNRI | 2678 | 1.01% |
| EWIREQLRPALKERLP | 2354 | 0.88% |
| SFFWNKLRYHLPAPQH | 2297 | 0.86% |
| LQNQWKLALTKVANLV | 2285 | 0.86% |
| VEPTQVRKQSGAVENA | 2283 | 0.86% |
| DHHLDLPRGKAIKPEILG | 2280 | 0.86% |
| HQAFWNRLWAKLKPPT | 1932 | 0.73% |
| NELVHVWSSKANSLVG | 1836 | 0.69% |
| GFLYQAFAREGFKIFM | 1764 | 0.66% |
| YLKWGYPWFWDRLRFA | 1680 | 0.63% |
| LVAHEISKKMKIFATI | 1671 | 0.63% |

**[Table 3]**

| **P8_MyD88** | | |
|---|---|---|
| **Peptide sequence** | **NGS count** | **Population** |
| DPQDLDWFYERLVSLF | 54757 | 21.10% |
| LGYHWIDWEYFVGIED | 25216 | 9.72% |
| DWVWDYLLPNLEGPAG | 19394 | 7.47% |
| WALDELLQILHARELF | 14404 | 5.55% |
| DPQDLDWFYERLVSLL | 13137 | 5.06% |
| PQDLEWFYERLVSLIL | 10568 | 4.07% |
| WALDELLKILYANETM | 10515 | 4.05% |
| DSLFWDRLLYSLHVTE | 7586 | 2.92% |
| WDFASNMEQFWDRLYY | 7352 | 2.83% |
| YLLLSVFQTIFARRTE | 7165 | 2.76% |
| YASYDDNDYYWVTHVLLRHL | 5893 | 2.66% |
| DPQDLQWFYERLVSLI | 5730 | 2.21% |
| SAAYDDFWDRLIRMIE | 5335 | 2.06% |
| VQWDDPWFWDKLAYAM | 4902 | 1.89% |
| DPQDLEWFYERLFSQM | 4488 | 1.73% |
| DYASSTWWLRELLQIA | 4111 | 1.58% |
| YLRADDSWFWEKLLYA | 4067 | 1.57% |
| YAASPWWFDELVKIVG | 3433 | 1.32% |
| HWIDWEYFVGIEDHIK | 3422 | 1.32% |
| IDDDHLTYLVRLLLSR | 3059 | 1.18% |
| YASSSWALDELLRILY | 2327 | 0.90% |
| VANILGYHWIDWEYFV | 2029 | 0.78% |
| GLSWGPYEWWMGLYDA | 1965 | 0.76% |
| SPQEQDGFWVRLIEAL | 1842 | 0.71% |
| QLIQQWIELHLHQLHL | 1605 | 0.62% |
| DWVWEHFFPMEEKDQT | 1538 | 0.59% |
| MFGGDTFHDFLDLISM | 1489 | 0.57% |
| LEANDSLFWDRLLYSL | 1465 | 0.56% |

**[Table 4]**

| **P3_MyD88** | | |
|---|---|---|
| **Peptide sequence** | **NGS count** | **Population** |
| SSFREGLWRLLELSKK | 79510 | 23.15% |
| NQWRNALTAAANLSGW | 28662 | 8.35% |
| DHHLDLPRGKAIKPEILG | 26722 | 7.78% |
| YTALTNSGFHTFRDND | 17600 | 5.12% |
| LVAHEISKKMKIFATI | 17452 | 5.08% |
| LKVESWRQALEKAGKL | 16847 | 4.91% |
| EWIREQLRPALKERLP | 16821 | 4.90% |
| YAFTGHLHRYLVQRGV | 13420 | 3.91% |
| NELVHVWSSKANSLVG | 8816 | 2.57% |
| KNLFNELVSKNIRTFM | 8543 | 2.49% |
| LKWGDPYFWSKLRYAM | 8310 | 2.42% |
| YLKWGYPWFWDRLRFA | 7321 | 2.13% |
| SENYATSPWALDELVL | 5494 | 1.89% |
| KEVEGWRDALTKVASL | 5187 | 1.80% |
| SELRVVDDGSVSPLEM | 4872 | 1.42% |
| AVDKETDKIQTPLKNA | 4790 | 1.39% |
| NGHESEIIKEIVDTIV | 4233 | 1.23% |
| FLSYRALEKRFGPIGQ | 3289 | 0.96% |
| YNFISHLYDALHRNRI | 2891 | 0.84% |
| GFLYQAFAREGFKIFM | 2579 | 0.75% |
| YYLDPSHLRKQSGEFG | 2538 | 0.74% |
| PEKDNGGENAFINNIV | 2498 | 0.73% |
| DTKLKKGESIAPELLR | 2465 | 0.72% |
| RKGDLISDELLIAIEE | 2452 | 0.71% |
| HLLKEFDKKLITAFKD | 2399 | 0.70% |
| PSEVRKQTGSLAKAFA | 2253 | 0.66% |
| TSHLHHALRLNKIETF | 2076 | 0.60% |
| ESILNQLRRSSQSIAN | 1976 | 0.58% |

In addition, to confirm the efficacy of the peptide, a sequence (CPP; RQIKIWFQNRRMKWKK, SEQ ID NO: 23) for cell membrane penetration was synthesized by fusion to the above-listed sequences, and peptides consisting of the sequences in Table 5 were manufactured.

**[Table 5]**

| **Synthetic peptide** | **Peptide sequence** (Cell penetrating peptide + TIR-binding motif) | | **SEQ ID NO:** |
|---|---|---|---|
| **TDIP1** | RQIKIWFQNRRMKWKK | WFYERLVSLILRDTKV | SEQ ID NO: 1 |
| **TDIP2** | RQIKIWFQNRRMKWKK | AFLYTFLINLTHPTNF | SEQ ID NO:2 |
| **TDIP3** | RQIKIWFQNRRMKWKK | AFFWAQLKSVLGNPTM | SEQ ID NO: 3 |
| **TDIP4** | RQIKIWFQNRRMKWKK | RNFVAYLFQALANKGI | SEQ ID NO: 4 |
| **TDIP5** | RQIKIWFQNRRMKWKK | TFLGHLYNALVQAGIH | SEQ ID NO: 5 |
| **TDIP6** | RQIKIWFQNRRMKWKK | LFFWESLKQLLRSDGR | SEQ ID NO: 6 |
| **TDIP7** | RQIKIWFQNRRMKWKK | YAFTGHLHRYLVQRGV | SEQ ID NO: 7 |
| **TDIP8** | RQIKIWFQNRRMKWKK | SSFREGLWRLLELSKK | SEQ ID NO: 8 |
| **TDIP9** | RQIKIWFQNRRMKWKK | PNLRTKVLNYLMRTKT | SEQ ID NO: 9 |
| **TDIP10** | RQIKIWFQNRRMKWKK | NQWRNALTAAANLSGW | SEQ ID NO: 10 |
| **TDIP11** | RQIKIWFQNRRMKWKK | RGFLSHLRHHLQAKDH | SEQ ID NO: 11 |
| **TDIP12** | RQIKIWFQNRRMKWKK | KEVEGWRDALTKVASL | SEQ ID NO: 12 |
| **TDIP13** | RQIKIWFQNRRMKWKK | DPQDLDWFYERLVSLF | SEQ ID NO: 13 |
| **TDIP14** | RQIKIWFQNRRMKWKK | LGYHWIDWEYFVGIED | SEQ ID NO: 14 |
| **TDIP15** | RQIKIWFQNRRMKWKK | DWVWDYLLPNLEGPAG | SEQ ID NO: 15 |
| **TDIP16** | RQIKIWFQNRRMKWKK | WALDELLQILHARELF | SEQ ID NO: 16 |
| **TDIP17** | RQIKIWFQNRRMKWKK | DPQDLDWFYERLVSLL | SEQ ID NO: 17 |
| **TDIP18** | RQIKIWFQNRRMKWKK | DHHLDLPRGKAIKPEILG | SEQ ID NO: 18 |
| **TDIP19** | RQIKIWFQNRRMKWKK | YTALTNSGFHTFRDND | SEQ ID NO: 19 |
| **TDIP20** | RQIKIWFQNRRMKWKK | LVAHEISKKMKIFATI | SEQ ID NO: 20 |
| **TDIP21** | RQIKIWFQNRRMKWKK | EWIREQLRPALKERLP | SEQ ID NO: 21 |
| **CP (Negative control)** | RQIKIWFQNRRMKWKK | GGAASSTTGGSSAATT | SEQ ID NO: 22 |

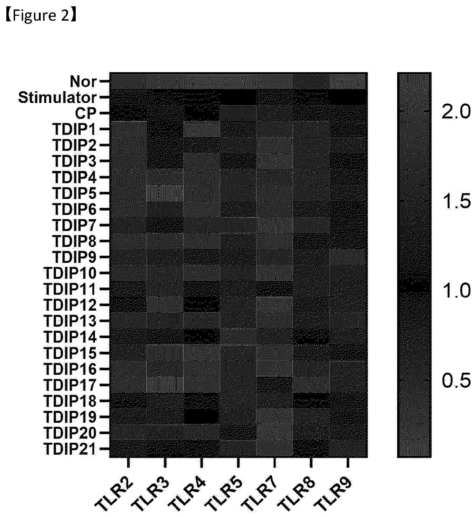

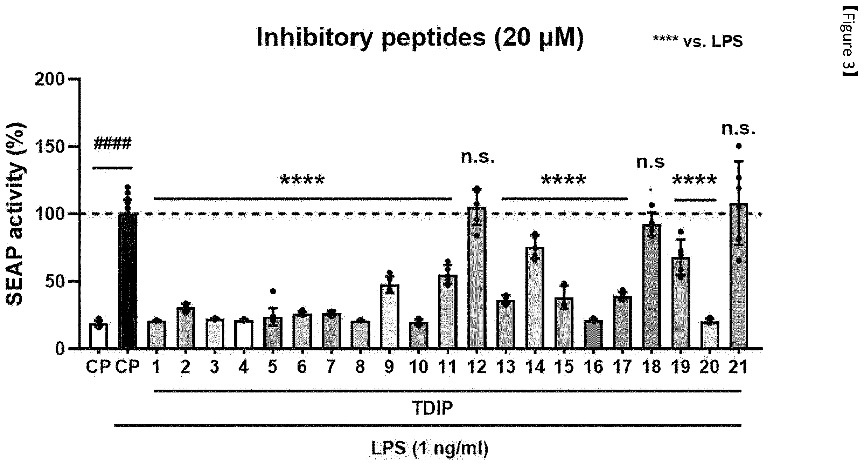

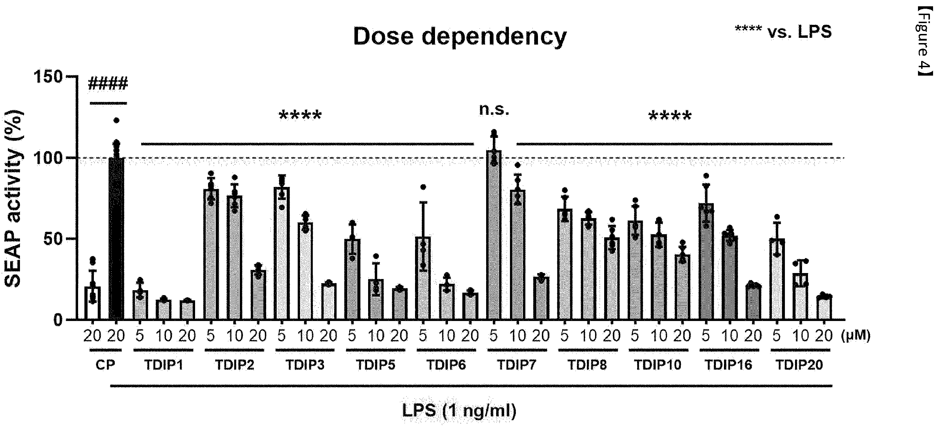

As a result of confirming the inhibitory efficacy of 21 types of synthetic peptides on TLRs (TLR2, TLR3, TLR4, TLR 5, TLR7, TLR8, and TLR9) using TLR-overexpressing HEK-Blue hTLR2, 3, 4, 5, 7, 8, and 9 reporter cell lines for the screened peptides shown in Tables 1, 2, 3, and 4, most of the peptides showed TLR2, TLR4, TLR7, and TLR9 inhibitory activity. In addition, some peptides showed inhibitory activity against TLR3 and TLR5 (FIG. 2). In addition, as shown in FIG. 3, as a result of confirming the inhibitory efficacy against TLR4 through SEAP reporter assay using HEK-Blue hTLR4, TLR4 inhibitory activity was confirmed in most of the peptides compared to the control. In addition, as shown in FIG. 4, as a result of confirming whether the TLR4 inhibitory activity was changed according to the concentration of each peptide (TDIP1, TDIP2, TDIP3, TDIP5, TDIP6, TDIP7, TDIP8, TDIP10, TDIP16, or TDIP20), it was confirmed that inhibitory activity was increased in a concentration-dependent manner in all peptides that were subjects of the experiment.

### 2-3. Verification of immunomodulatory efficacy using mouse immune cells

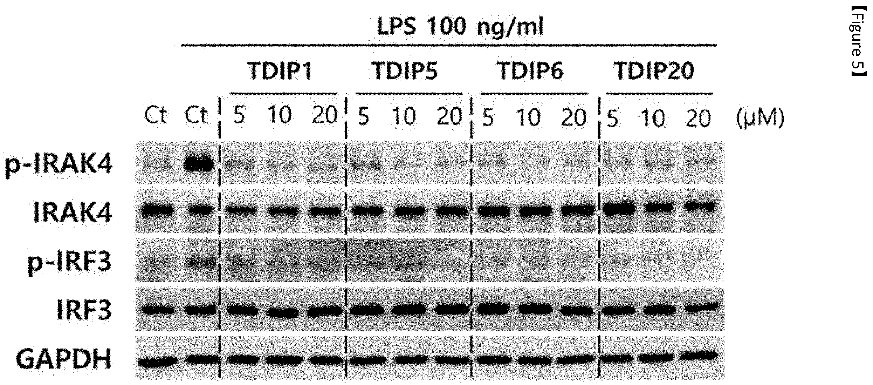

To verify the TLR4 inhibitory effect of TDIP1, TDIP5, TDIP6, and TDIP20 peptides among the above peptides, when mouse immune cells (macrophages, RAW264.7) were treated with the peptides after LPS treatment, the effect of inhibiting IRAK4 phosphorylation and IRF3 phosphorylation was confirmed. As a result, as shown in FIG. 5, it was able to be confirmed that the peptides of the present invention inhibits both MyD88- and TRIF-dependent TLR4 signaling.

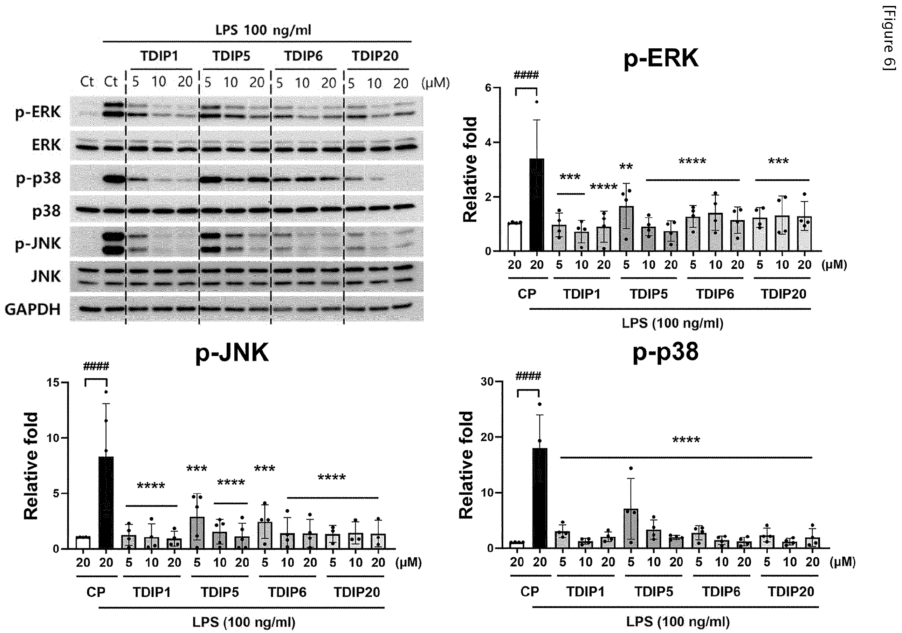

In addition, the effect of inhibiting TRL4-dependent MAPK signaling was confirmed. RAW264.7 cells were treated with the peptides in a concentration-dependent manner, and the ERK, JNK and p38 phosphorylation inhibitory effects in the treatment groups and a non-treatment group were confirmed. As a result, as shown in FIG. 6, when the peptides of the present invention were treated, it was confirmed that the phosphorylation of proteins involved in the MAPK signaling pathway is significantly reduced.

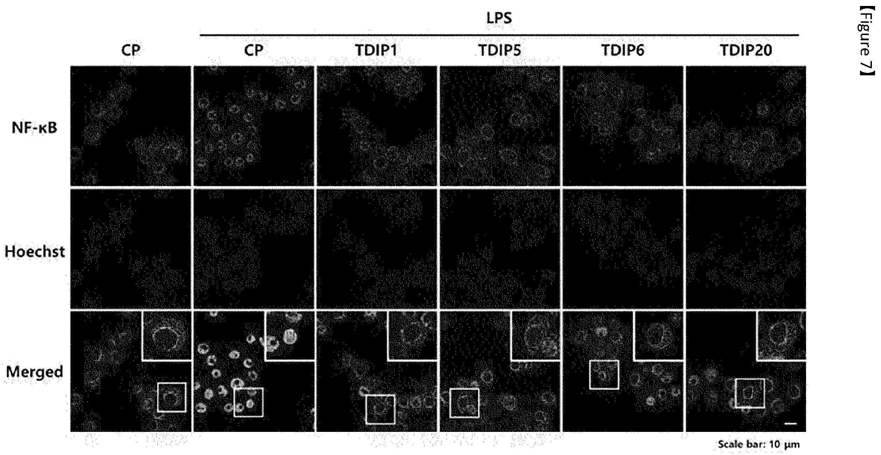

In addition, it was confirmed whether the peptides affect the intranuclear translocation of NF-kB. Mouse immune cells were treated with each of the four types of peptides, and then effects on the nuclear translocation of NF-kB were confirmed. As a result, as shown in FIG. 7, it can be seen that the intranuclear translocation of NF-kB was inhibited when the peptides were treated.

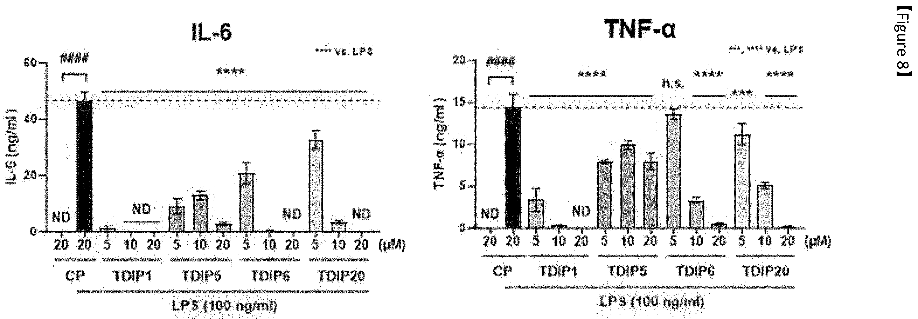

In addition, it was confirmed that treatment with the above peptides inhibits cytokine secretion in mouse macrophages. It was confirmed that IL-6 and TNF-α secretion levels in mouse macrophages tend to be reduced by treatment with the above peptides, compared to the control (FIG. 8).

### Example 3. Confirmation of sepsis treatment effect of peptides with TLR inhibitory activity

Experiments were conducted on mice to confirm that the peptides with TLR inhibitory activity of the present invention are effective in treating sepsis. Each of the peptides (TDIP1, TDIP5, TDIP6, and TDIP20) with TLR inhibitory activity of the present invention was intraperitoneally injected into mice with sepsis induced by LPS at a dose of 10 nmol per g of mouse weight, and ELISA was performed to confirm a mouse survival rate and the expression levels of cytokines (IL-23, IL-1α, IFN-γ, TNF-α, MCP-1, IL-12p70, IL-1β, IL-10, IL-6, IL-27, IL-17A, and IFN β) in serum 16 hours after LPS and peptide administration. A TUNEL assay was performed to confirm cell death in renal tissue.

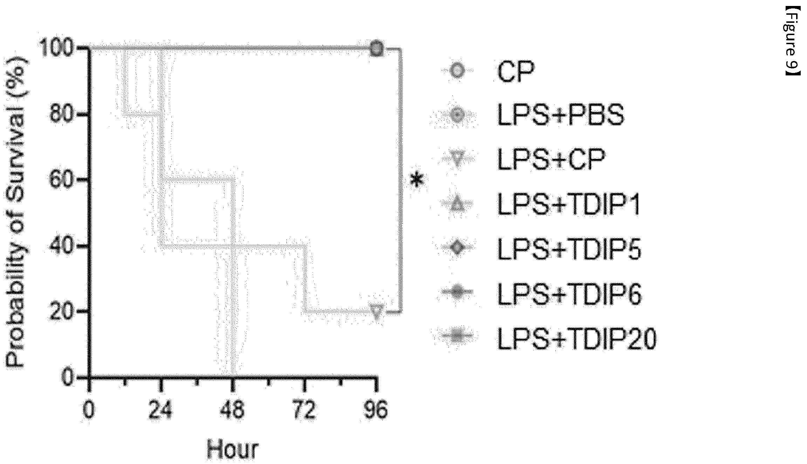

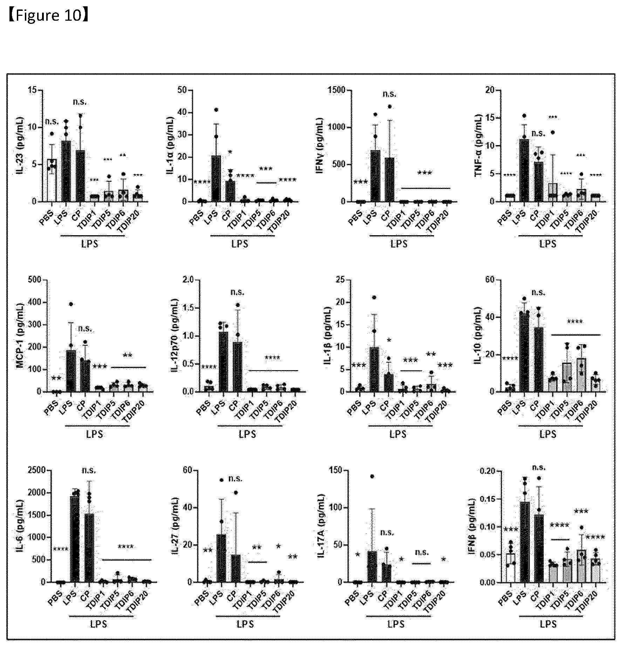

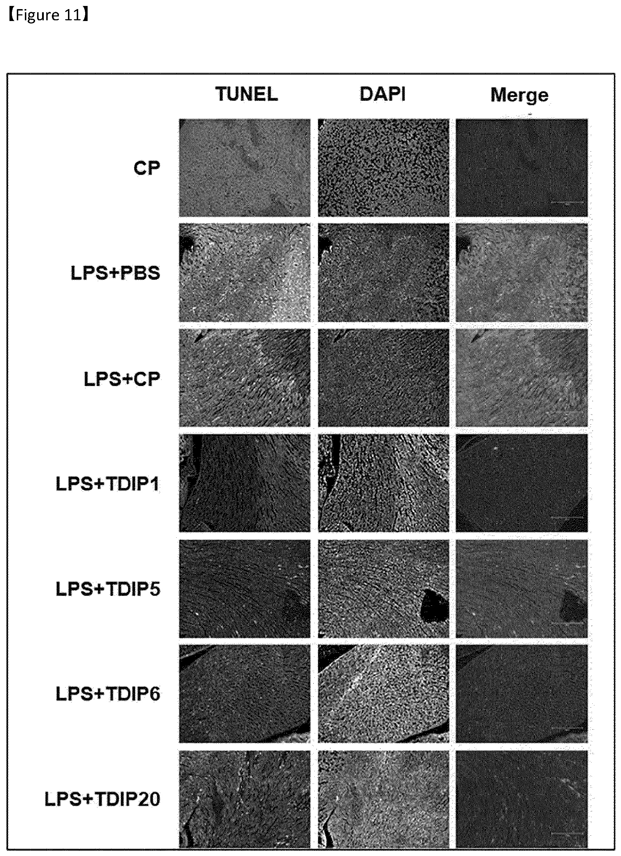

As a result, as shown in FIG. 9, it was confirmed that the survival rate of the peptide-treated diseased mice was greatly increased as compared with that of a non-administration group and a CP-administration group, which died between 48 to 96 hours. In addition, as shown in FIG. 10, it can be seen that the secretion amount of the cytokines that increased in sepsis-induced mice was mostly reduced by administration of the peptides with TLR inhibitory activity of the present invention. In addition, in the TUNEL assay for confirming cell death in renal tissue, TUNEL positive cells increased in a non-administration group and a CP-administration group, whereas the TUNEL positive cells decreased in the peptide-administration groups of the present invention, indicating decreased cell death (FIG. 11).

### Example 4. Confirmation of dry macular degeneration treatment effect of peptides with TLR inhibitory activity

To confirm that the peptides with TLR inhibitory activity of the present invention are effective in treating dry macular degeneration, each of the peptides (TDIP1,TDIP5, TDIP6, and TDIP20) with TLR inhibitory activity of the present invention was directly administered once a day for 21 days at a concentration of 20 µM (5 µL) to the upper corneal surface of a mouse model in which dry macular degeneration was induced by intraperitoneal administration of NaIO₃, and then the thicknesses of the outer nuclear layer (ONL) and the retinal pigment epithelium (RPE) of the diseased mouse and the effect on retinal cell death were confirmed.

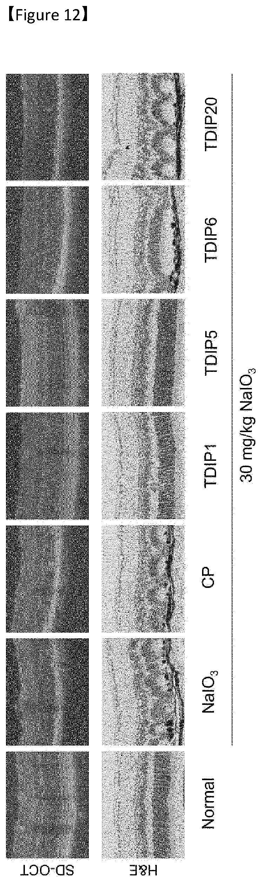

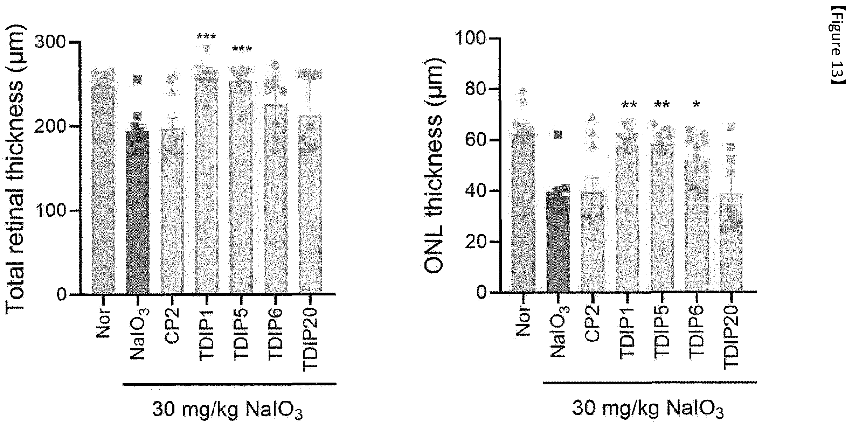

As a result, as shown in FIGS. 12 and 13, when dry macular degeneration was induced by NaIO₃, it was confirmed that waste products increased and thicknesses of the RPE and the ONL decreased, but the thicknesses were maintained in the TDIP1-, TDIP5-, and TDIP6-administration groups without waste products.

In the above, the present invention was described with reference to embodiments. It will be understood by those of ordinary skill in the art that the present invention can be implemented in modified forms without departing from the essential features of the present invention. Therefore, the disclosed embodiments should be considered from a descriptive perspective, rather than a restrictive perspective. The scope of the present invention is shown in the claims rather than the foregoing description, and all differences within the equivalent range thereto will be construed as being included in the present invention.

### [Modes of the Invention]

As one aspect of the present invention, the present invention relates to a method of constructing a phage display library of TIR domain surface sequence-derived peptide fragments, which includes manufacturing peptide fragments having a length of 16 to 21 amino acids, in which a partial sequence overlaps a sequence exposed on the surface involved in interdomain binding, excluding the core region of the tertiary structure of TIR domains derived from various species of organisms; synthesizing DNA encoding each of the peptide fragments; cloning the synthesized DNA using a phagemid vector; and constructing a library for phage display by introducing the cloned vector into a bacteriophage.

In one embodiment of the present invention, the overlapping sequence of the peptide fragments has a length of 5 to 8 amino acids.

As another aspect of the present invention, the present invention relates to a method of screening peptides with TLR signaling inhibitory activity, which includes screening peptides specifically binding to TIR domains including human MAL or MyD88 from the phage display library constructed by the above-described method.

In one embodiment of the present invention, the peptides with TLR signaling inhibitory activity specifically bind to TIR domains included in humans, bacteria, or plants, including human MAL or MyD88, to suppress binding between TIR domains, thereby inhibiting toll-like receptor (TLR) signals.

As still another aspect of the present invention, the present invention relates to a TLR signal-inhibiting peptide, which includes a peptide fragment derived from a surface sequence of a TIR domain.

In one embodiment of the present invention, the TLR may be selected from the group consisting of TLR2, TLR3, TLR4, TLR5, TLR7, and TLR9.

In one embodiment of the present invention, the peptide is screened by the method of claim 4.

In one embodiment of the present invention, the peptide includes one or more selected from peptides consisting of the amino acid sequences of SEQ ID NOs: 1 to 21.

In one embodiment of the present invention, the peptide is fused with the peptide consisting of SEQ ID NO: 23.

As yet another aspect of the present invention, the present invention relates to a pharmaceutical composition for preventing, improving, or treating an inflammatory disease, which includes the peptide of claim 5.

In one embodiment of the present invention, the inflammatory disease may be selected from the group consisting of dermatitis, allergies, atopy, asthma, periodontitis, allergic and non-allergic rhinitis, otitis media, pharyngitis, tonsillitis, pneumonia, peptic ulcers, chronic and acute gastritis, Crohn's disease, colitis, chronic and acute enteritis, hemorrhoids, gout, ankylosing spondylitis, osteoarthritis, rheumatoid arthritis, periarthritis, tendonitis, tenosynovitis, myositis, acute and chronic hepatitis, Sjogren's syndrome, multiple sclerosis, rhinitis, chronic obstructive pulmonary disease, irritable bowel syndrome, viral infections, bacterial infections, fungal infections, burns, wounds from surgical or dental operations, atherosclerosis, arthritis, Hodgkin's disease, pancreatitis, iritis, scleritis, uveitis, conjunctivitis, dry macular degeneration, wet macular degeneration, sepsis, and septic shock.

In one embodiment of the present invention, the inflammatory disease may be sepsis, septic shock, symptoms associated with sepsis, or multiorgan dysfunction syndrome.

In one embodiment of the present invention, the inflammatory disease may be macular degeneration.

In one embodiment of the present invention, the macular degeneration may be dry macular degeneration.

In one embodiment of the present invention, the composition may reduce the thickness of the retinal pigment epithelium (RPE) or the outer nuclear layer (ONL).

In one embodiment of the present invention, the peptides may include one or more selected from peptides consisting of the amino acid sequences of SEQ ID NOs: 1, 5, 6, and 20.

## Claims

1. A method of constructing the phage display library of TIR domain surface sequence-derived peptide fragments, comprising:
manufacturing peptide fragments having a length of 16 to 21 amino acids, in which a partial sequence overlaps a sequence exposed on the surface involved in interdomain binding, excluding the core region of the tertiary structure of TIR domains derived from various species of organisms;
synthesizing DNA encoding each of the peptide fragments;
cloning the synthesized DNA using a phagemid vector; and
constructing a library for phage display by introducing the cloned vector into a bacteriophage.

2. The method of claim 1, wherein the overlapping sequence of the peptide fragment has a length of 5 to 8 amino acids.

3. A method of screening a peptide having TLR signaling inhibitory activity, comprising:
screening peptides that specifically bind to TIR domains including human MAL or MyD88 from the phage display library constructed by the method of claim 1.

4. The method of claim 3, wherein the peptides having TLR signaling inhibitory activity specifically bind to human TIR domains including human MAL or MyD88, or TIR-family domains in bacteria or plants to suppress binding between TIR domains, thereby inhibiting toll-like receptor (TLR) signaling.

5. A toll-like receptor (TLR) signal-inhibiting peptide, comprising a peptide fragment derived from a surface sequence of a TIR domain.

6. The peptide of claim 5, wherein the TLR is any one or more selected from the group consisting of TLR2, TLR3, TLR4, TLR5, TLR7, and TLR9.

7. The peptide of claim 5, wherein the peptide is screened by the method of claim 4.

8. The peptide of claim 5, wherein the peptide includes any one or more selected from peptides consisting of the amino acid sequences of SEQ ID NOs: 1 to 21.

9. The peptide of claim 5, wherein the peptide is fused with a peptide consisting of the sequence of SEQ ID NO: 23.

10. A pharmaceutical composition for preventing, improving or treating an inflammatory disease, comprising the peptide of claim 5.

11. The pharmaceutical composition of claim 10, wherein the inflammatory disease is selected from the group consisting of dermatitis, allergies, atopy, asthma, periodontitis, allergic and non-allergic rhinitis, otitis media, pharyngitis, tonsillitis, pneumonia, peptic ulcers, chronic and acute gastritis, Crohn's disease, colitis, chronic and acute enteritis, hemorrhoids, gout, ankylosing spondylitis, osteoarthritis, rheumatoid arthritis, periarthritis, tendonitis, tenosynovitis, myositis, acute and chronic hepatitis, Sjogren's syndrome, multiple sclerosis, rhinitis, chronic obstructive pulmonary disease, irritable bowel syndrome, viral infections, bacterial infections, fungal infections, burns, wounds from surgical or dental operations, atherosclerosis, arthritis, Hodgkin's disease, pancreatitis, iritis, scleritis, uveitis, conjunctivitis, dry macular degeneration, wet macular degeneration, sepsis, and septic shock.

12. The pharmaceutical composition of claim 10, wherein the inflammatory disease is sepsis, septic shock, symptoms associated with sepsis, or multiorgan dysfunction syndrome.

13. The pharmaceutical composition of claim 10, wherein the inflammatory disease is macular degeneration.

14. The pharmaceutical composition of claim 13, wherein the macular degeneration is dry macular degeneration.

15. The pharmaceutical composition of claim 13, wherein the composition reduces the thickness of the retinal pigment epithelium (RPE) or the outer nuclear layer (ONL).

16. The pharmaceutical composition of claim 10, wherein the peptide includes one or more selected from peptides consisting of the amino acid sequences of SEQ ID NOs: 1, 5, 6, and 20.
